# EUROPÄISCHE PATENTSCHRIFT

(11) **EP 2 758 012 B1**
(45) Veröffentlichungstag und Bekanntmachung des Hinweises auf die Patenterteilung: **15.11.2017**
(21) Anmeldenummer: 12773254.3
(22) Anmeldetag: 21.09.2012
(51) Int. Cl.: A61F 5/41

(54) **VAKUUMANSCHLUSS FÜR EIN PENISEXTENSIONSGERÄT**
VACUUM ATTACHMENT FOR A PENIS EXTENSION DEVICE
RACCORD À VIDE POUR UN APPAREIL D'EXTENSION DU PÉNIS

(30) Priorität: 22.09.2011 EP 11182330
(43) Veröffentlichungstag der Anmeldung: 30.07.2014
(60) Teilanmeldung: 16186189.3
(73) Patentinhaber: Swiss-Tec Global Limited, San Gwann SGN 3000 (MT)
(72) Erfinder: JOCHUM, Herbert, 82541 Münsing (DE)
(74) Vertreter: Köster, Hajo
(86) Internationale Anmeldenummer: PCT/EP2012/068640
(87) Internationale Veröffentlichungsnummer: WO 2013/041675

(56) Entgegenhaltungen:
- WO-A1-2004/004610
- WO-A1-2010/094677
- DE-A1-102007 017 222
- DE-U1- 8 700 174
- US-A- 5 707 341
- US-A1- 2007 093 687

## Beschreibung

Die Erfindung betrifft eine Verbindungsvorrichtung für den distalen Teil eines Penis mit einer Penisextensionsvorrichtung, wobei die Verbindungsvorrichtung einen am proximalen Ende offenen, länglichen, starren Hohlkörper, der zur Aufnahmen des distalen Endes des Penis und zur Befestigung an der Penisextensionsvorrichtung dient, und an seinem distalen Ende eine Öffnung besitzt, die in Wirkverbindung mit einer Vakuumpumpe steht oder in eine derartige Wirkverbindung gebracht werden kann,
und eine elastische, schlauchartige Manschette zur Anlage mit ihrem proximalen Bereich an den Penisschaft, die im zusammengebauten Zustand der Verbindungsvorrichtung mit ihrem distalen Bereich das proximale Ende des Hohlkörpers umschließt und außen an ihm anliegt, aufweist,

Penisextensionsgeräte bzw. -vorrichtungen sind in den verschiedensten Ausgestaltungen bekannt, man vergleiche beispielsweise DE 100 01 331 A1, US 5 707 341 und EP 1 779 822 A1.

Ein gattungsgemäßes tragbares Penisextensionsgerät ist auch in der DE 10 2007 017 222 A1 beschrieben.

Diesen Geräten ist gemeinsam, dass ein länger wirkender Zug auf den Penis ausgeübt wird, so dass sich neues Gewebe bildet und zu einer Vergrößerung des Penis führt. Mit diesen Vorrichtungen können auch bestimmte Erkrankungen behandelt werden. Es handelt sich hier somit nicht um Erektionshilfen oder Ähnliches.

Eine Erektionshilfe bzw. eine elektronische tragbare Aneurysma-Verstärkungsvorrichtung für einen Penis ist in der WO 2004/004610 A1 beschrieben, bei der mit Hilfe einer Pumpe Luft bewegt und dadurch eine Expansions- und Konzentrationsbewegung der Penismuskeln hervorgerufen wird.

Bei der hier in Rede stehenden Penisextensions-vorrichtung wird jedoch ein Zug auf den Penis im nicht erigierten Zustand ausgeübt. Dazu ist es erforderlich, die Penisextensionsvorrichtung mit dem Penis zu verbinden, damit dieser Zug ausgeübt werden kann. Selbstverständlicherweise sollte der Ansatzpunkt für die Zugübermittlung am distalen Ende des Penis erfolgen.

Es ist nun vorgeschlagen worden, eine Penisextensionsvorrichtung mit dem Penis mit Hilfe einer hinter der Eichel des Penis angreifenden Schlaufe zu verbinden. Ferner sind Kondome und zylinderförmige Hohlkörper (US 5 707 341 und EP 1 779 822 A1) vorgeschlagen worden.

Aus der DE 20 206 017 667 U1 und DE 20 207 003 824 U1 sind sogenannte Eichelschlitten bekannt, auf denen der Penis aufgelegt und mit Hilfe eines Fixierelementes befestigt wird. Das Fixierelement greift hinter der Eichel und somit an der proximalen Seite der Eichel an und verhindert, dass der Penis beim Ausüben eines Zugs von dem Auflageelement weggezogen wird.

Nachteilig an diesen bekannten Befestigungselementen ist, dass sie entweder den Penis nicht verlässlich fixieren und/oder umständlich anzulegen sind.

Aufgabe der Erfindung ist es, eine einfach aufgebaute gattungsgemäße Verbindungsvorrichtung bereitzustellen, die einfach aufgebaut ist und eine verlässliche sowie schützende Verbindung zur Penisextensionsvorrichtung gewährleistet.

Gelöst wird diese Aufgabe durch eine erfindungsgemäße Verbindungsvorrichtung gemäß Anspruch 1.

Die erfindungsgemäße Verbindungsvorrichtung ist dadurch gekennzeichnet, dass zwischen der Öffnung und der Vakuumpumpe (dem Innenraum) ein Drei-Wege-Ventil angeordnet ist, das folgende Stellungen einnehmen kann:
i) Der Innenraum des Hohlkörpers ist mit der Umwelt bzw. der Außenwelt verbunden; dies stellt die Belüftungsstellung dar;
ii) Die Öffnung und somit der Hohlkörper sind verschlossen; dies stellt die Verschlussstellung dar; und
iii) der Innenraum des Hohlkörpers ist mit der Vakuumpumpe (genauer dem Inneren der Vakuumpumpe) verbunden; dies stellt die Pumpstellung dar.

Das Drei-Wege-Ventil weist vorzugsweise eine drehbare Dichtungsscheibe auf, in der mindestens eine durchgehende Öffnung vorgesehen ist, die dezentral angeordnet ist und durch Drehen der Dichtungsscheibe in axiale Ausrichtung mit der Öffnung gebracht werden kann. Diese Stellung stellt die Pumpstellung dar.

In der Dichtungsscheibe ist weiterhin eine weitere durchgehende Öffnung vorgesehen, die ebenfalls dezentral angeordnet ist und durch Drehen der Dichtungsscheibe in axiale Ausrichtung mit der Öffnung gebracht werden kann und die über einen Kanal mit der Umwelt in Verbindung steht. Bei dieser Stellung handelt es sich um die Belüftungsstellung.

Ferner kann die Dichtungsscheibe derart gedreht werden, dass die Öffnung verschlossen ist. Bei dieser Stellung handelt es sich um die Verschlussstellung.

Vorzugsweise bilden das Dreiwegeventil oder bilden Elemente des Dreiwegeventils zusammen mit der Vakuumpumpe eine zusammen handhabbare Einheit, die weiterhin vorzugsweise von dem Hohlkörper vollständig entfernt und auch wieder damit zusammengesetzt werden kann.

Die Querschnittsform des Hohlkörpers kann beliebig sein. Nach einer weiterhin bevorzugten Ausführungsform ist der Hohlkörper zylinderförmig ausgebildet und stellt zumindest im proximalen Bereich eine Art Hohlzylinderrohr dar. Es muss sich dabei nicht um einen exakten Zylinder handeln; so kann die Querschnittsform des Hohlkörpers nicht nur kreisförmig sondern auch elliptisch sein.

Die erfindungsgemäße Verbindungsvorrichtung kann mit einer wie nachstehend näher beschriebenen erfindungsgemäßen Manschette oder einer üblichen Manschette zusammenwirken.

Nach einer weiterhin bevorzugten Ausführungsform ist der Hohlkörper an seinem proximalen Rand mit einem sich nach radial außen erstreckenden Wulst ausgestattet bzw. bildet einen solchen Wulst. Die Manschette erstreckt sich im Übergangsbereich wulstartig nach radial außen und weist in ihrer Innenwandung eine umlaufende Rinne auf, deren Boden nach radial außen zeigt und die in etwa, bezogen auf den Querschnitt der Rinne bzw. des Wulstes, derart flächen- und formkongruent zum Wulst des Hohlkörpers ist, dass der Wulst des Hohlkörpers in der Rinne zu liegen kommen kann und dort verbleibt.

Der Hohlkörper ist an seinem distalen Ende offen. Bei dem Hohlkörper kann es sich somit im einfachsten Fall um eine Art Hohlzylinderrohr handeln. Die Öffnung steht vorzugsweise in Wirkverbindung mit einer Vakuumpumpe. Dadurch kann Unterdruck im Inneren des Hohlkörpers erzeugt werden, wenn die Manschette abdichtend gegen den Penisschaft und den Hohlkörper anliegt. Natürlich sind dann geeignete Maßnahmen zu ergreifen, dass dieses Vakuum erhalten bleibt, beispielsweise indem ein Rückschlagventil vorgesehen wird. Durch dieses Vakuum bleibt die erfindungsgemäße Verbindungsvorrichtung dauerhafter mit dem Penisschaft verbunden, selbst wenn durch ein übliches Penisextensionsgerät ein Zug auf die erfindungsgemäße Verbindungsvorrichtung ausgeübt wird.

Der starre Hohlkörper besteht aus einem geeigneten Kunststoff und ist zweckmäßigerweise spritzgeformt. Unter einem starren Hohlkörper werden erfindungsgemäß nicht nur solche Hohlkörper verstanden, die überhaupt nicht verformbar sind. Vielmehr sind auch solche Hohlkörper mit umfasst, die unter Druck oder Zug eine gewisse bzw. leichte Verformbarkeit besitzen, jedoch die ihnen gegebene Form im Wesentlichen beibehalten und somit über eine ausreichende Formstabilität verfügen.

Die Zugkraft, welche von dem Penisextensionsgerät auf die Verbindungsvorrichtung ausgeübt wird, kann durch ein geeignetes Kupplungselement, welches mit dem Hohlkörper auf beliebige Weise verbunden ist, ausgeübt werden.

Gegenstand der Erfindung ist auch eine elastische schlauchartige Manschette für eine erfindungsgemäße Verbindungsvorrichtung zur Anlage mit ihrem proximalen Bereich an den Penisschaft, wobei die Wanddicke der Manschette im distalen Bereich größer ist als im proximalen Bereich, die dadurch gekennzeichnet ist, dass die Wanddicke der Manschette im distalen Bereich 4 bis 15 mal so stark ist wie die Wanddicke der Manschette im proximalen Bereich.

Auf diese Weise wird erreicht, dass die Manschette im proximalen Bereich derart gewählt ist, dass die Manschette, welche eine Art Kondom darstellt, in diesem Bereich, der an den Penisschaft zu liegen kommt, ausreichend flexibel ist. Im distalen Bereich verfügt die Manschette aufgrund der stärkeren Wanddicke über eine ausreichende Eigenstabilität und Belastbarkeit, sodass die auf die Manschette in diesem Bereich ausgeübten Kräfte aufgenommen werden können. Gleichzeitig ist sie jedoch noch elastisch genug, um auf den starren Hohlkörper aufgebracht werden zu können. Zudem behält sie ihre eigene Form im Wesentlichen bei.

Die Wanddicke der Manschette im distalen Bereich ist 4 bis 15 mal so stark ist wie die Wanddicke der Manschette im proximalen Bereich. Mit der Angabe "4 bis 15 mal so stark" sind alle in diesen Bereich fallenden Werte offenbart, insbesondere 4 mal, 5 mal, 6 mal, 7 mal, 8 mal, 9 mal, 10 mal, 11 mal, 1 mal, 13, mal, 14 mal und 15 mal so stark.

Vorzugsweise beträgt die Wanddicke der Manschette im distalen Bereich 0,20 bis 0,40 mm und im distalen Bereich 2 bis 4 mm.

Der genannte Bereich 0,20-0,40 mm für die Wanddicke der Manschette im distalen Bereich umfasst alle in dem genannten Bereich liegende Werte, beispielsweise 0,20, 0,21, 0,22, 0,23, 0,24, 0,25, 0,26, 0,27, 0,28, 0,29, 0,30, 0,31, 0,32, 0,33, 0,34, 0,35, 0,36, 0,37, 0,38, 0,39 und 0,40 mm. Insbesondere bevorzugte Werte sind 0,25, 0,30 und 0,35 mm.

Der genannte Bereich von 2 bis 5 mm für die Wanddicke der Manschette im distalen Bereich umfasst alle in diesen Bereich fallenden Werte, beispielsweise 2,0, 2,5, 3,0 3,5, 4,0, 4,5 und 5,0 mm. Insbesondere bevorzugt sind 2,5, 3,0 und 3,5 mm.

Nach einer bevorzugten Ausführungsform verringert sich die Wanddicke der Manschette am Übergang vom distalen Bereich zum proximalen Bereich hin kontinuierlich.

Der distale Bereich der Manschette ist vorzugsweise unterteilt in einen Übergangsbereich zum proximalen Bereich und einen dem Hohlkörper zugewandten Endbereich. Die Manschette ist im proximalen Bereich und in dem dem Hohlkörper zugewandten Bereich vorzugsweise zylinderförmig ausgebildet und stellt somit ebenfalls eine Art Hohlzylinderrohr dar. Aufgrund der geringen Wandstärke behält die Manschette im proximalen Bereich natürlich ihre Form ohne Unterstützung nicht bei, sondern "fällt zusammen", wie das bei üblichen Kondomen der Fall ist. Im distalen Bereich verfügt die Manschette jedoch aufgrund der größeren Wandstärke über eine ausreichende Formstabilität, um die Zylinderrohrform im ungestützten Zustand in etwa beizubehalten

Der Innendurchmesser der Manschette bezieht sich auf den hohlzylindrischen Zustand und ist im proximalen Bereich sowie in dem dem Hohlkörper zugewandten Endbereich in etwa der gleiche. Dies bezieht sich auf einen nichtgedehnten Zustand der verschiedenen Bereiche der Manschette. Der Innendurchmesser der Manschette im Übergangsbereich ist vorzugsweise größer, worauf nachstehend noch näher eingegangen wird.

Die Manschette wird vorzugsweise aus medizinischem Silikon gefertigt. Zweckmäßigerweise wird ein sehr weiches Silikon eingesetzt, das über eine 300 bis 500-fache Dehnbarkeit verfügt.

Die Erfindung wird im Folgenden anhand bevorzugter Ausführungsformen darstellenden, nicht maßstabsgetreuen sowie skizzenhaften Zeichnungen näher erläutert. In diesen Zeichnungen zeigen:
- Fig. 1: eine Schnittansicht einer erfindungsgemäßen Verbindungsvorrichtung mit einer erfindungsgemäßen Manschette, einem Hohlkörper und einer Vakuumpumpe,
- Fig. 2: eine Explosionsdarstellung der in der Fig. 1 gezeigten Verbindungsvorrichtung,
- Fig. 3: eine Seitenansicht der Manschette der in der Fig. 1 gezeigten Verbindungsvorrichtung,
- Fig. 4: eine Schnittansicht gemäß der Linien A-A der in der Fig. 3 gezeigten Manschette,
- Fig. 5: eine Längsschnittansicht einer weiteren Ausführungsform einer erfindungsgemäßen Verbindungsvorrichtung,
- Fig. 6: die in der Fig. 5 gezeigte weitere Ausführungsform in Explosionsdarstellung sowie im Längsschnitt,
- Fig. 7: eine perspektivische Ansicht des Hohlkörpers der in der Fig. 5 gezeigten Ausführungsform, wobei zusätzlich eine drehbare Scheibe dargestellt ist, die ein Element eines Dreiwegeventils darstellt,
- Fig. 8: zwei Aufsichten auf die in der Fig. 7 gezeigte Scheibe mit zwei Schnitten A-A und B-B und
- Fig. 9: eine weitere Ausführungsform einer Verbindungsvorrichtung in Explosionsdarstellung, jedoch ohne eine Manschette.

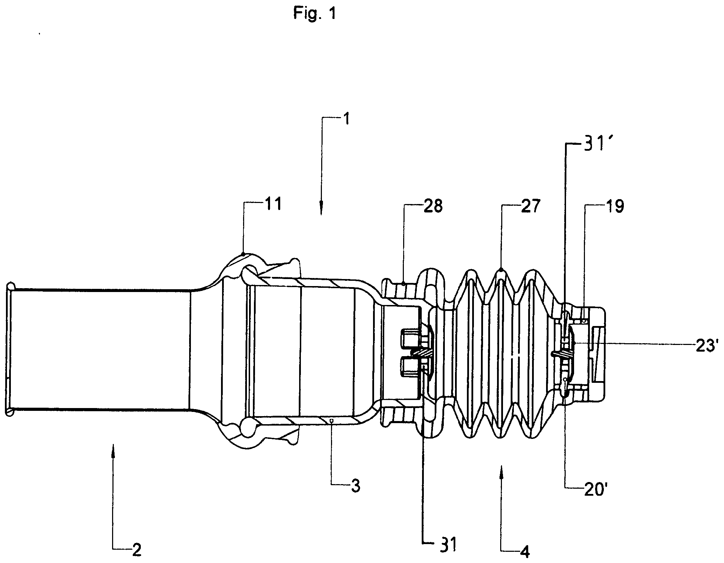

Die in der Fig. 1 gezeigte erfindungsgemäße Verbindungsvorrichtung 1 besitzt eine Manschette 2 aus medizinischem Silikon, die mit einem starren Hohlkörper 3 verbunden ist, welcher wiederum mit einer Vakuumpumpe 4 verbunden ist.

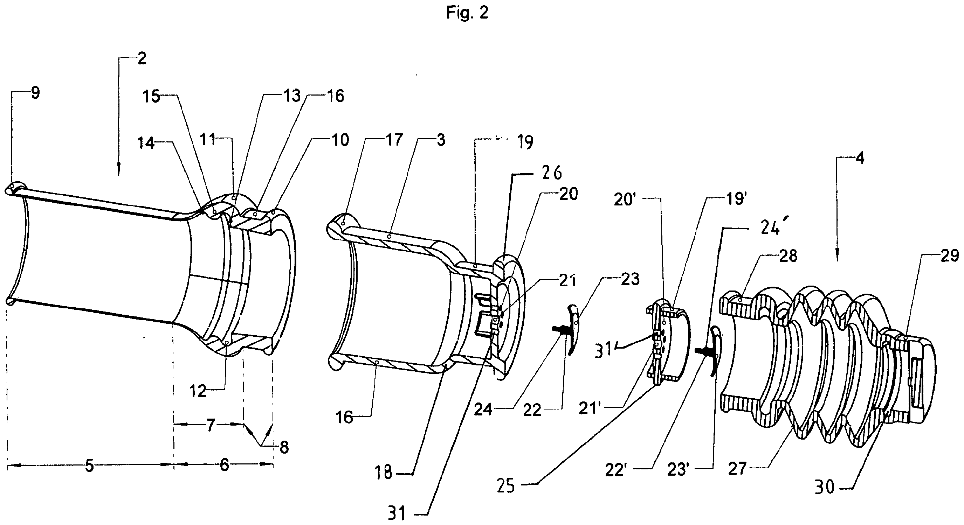

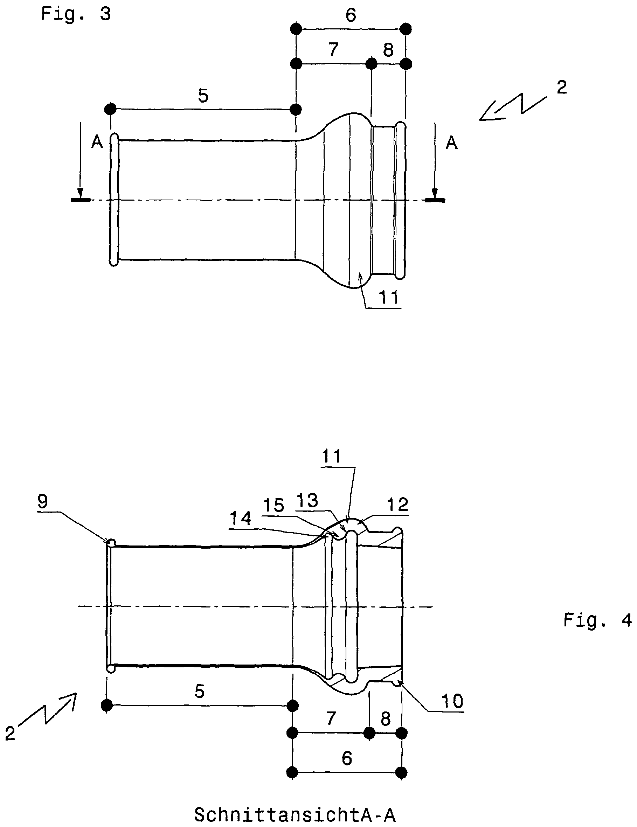

Wie insbesondere aus den Figuren 2 und 4 ersichtlich ist, weist die Manschette 2 zwei Bereiche auf, nämlich einen proximalen Bereich 5 und einen distalen Bereich 6. Der distale Bereich 6 ist wiederum unterteilt in einen Übergangsbereich 7 zum proximalen Bereich 5 und in einen zum Hohlkörper 3 zugewandten Endbereich 8.

Der proximale Bereich 5 der Manschette 2 kommt wie bei einem üblichen Kondom an dem Penisschaft zu liegen. Der proximale Rand 9 ist wulstartig ausgebildet und unterstützt somit die Aufrollbarkeit bzw. die Abrollbarkeit des proximalen Bereiches 5. Typische Durchmesserwerte für den proximalen Bereich 5, wenn dieser in zylindrischer Form vorliegt, betragen beispielsweise 20, 25 oder 30 mm.

Der Endbereich 8 des distalen Bereichs 6 der Manschette 2 besitzt ebenfalls einen sich nach radial außen erstreckenden Wulst 10. Ausgehend von diesem Wulst 10 erstreckt sich der Endbereich 8 in Richtung des proximalen Bereichs in hohlzylindrischer Form. Der Außendurchmesser dieses Endbereichs 8 bis zum Beginn des Übergangsbereichs 7 bleibt in etwa gleich. Gleiches gilt für den Innendurchmesser ausgehend vom Wulst/Rand 10 zum Übergangsbereich 7 hin. Die Wandstärke des Endbereichs 8 beträgt in etwa 3 mm.

Im Übergangsbereich 7 vergrößert sich der Außendurchmesser beginnend beim Endbereich 8 um einige Millimeter. Danach verringert sich der Außendurchmesser des Übergangsbereichs 7 in Richtung des proximalen Bereichs wieder und entspricht am Ende des Übergangsbereichs demjenigen des proximalen Bereichs 5. Die Manschette 2 bildet somit im Übergangsbereich 7 einen sich nach radial außen erstreckenden Wulst 11.

In demjenigen Abschnitt, in dem der Wulst 11 seinen größten Außendurchmesser besitzt, ist in der Innenwandung 13 eine radial umlaufende Rinne 12 ausgebildet. Der Boden der Rinne 12 zeigt nach radial außen. Der Innendurchmesser des Übergangsbereichs 7 der Manschette 2 im Bereich dieser Rinne 12 ist somit größer als der Innendurchmesser im Endbereich 8 und auch als der Innendurchmesser im proximalen Bereich 5.

Eine zweite Rinne 14 ist zwischen der Rinne 12 und dem proximalen Bereich 5 im Übergangsbereich 7 ausgebildet. Auch diese zweite Rinne 14 läuft radial um; ihr Boden zeigt ebenfalls nach radial außen. Zwischen den beiden Rinnen 12 und 14 ergibt sich bei dieser Konstruktion ein nach radial innen zeigender, umlaufender Wulst 15.

Beginnend bei diesem Wulst 15 verringert sich die Wandstärke der Manschette 2 im Übergangsbereich 7 und reduziert sich in proximaler Richtung beginnend bei der zweiten Rinne 14 soweit, bis sie die Wandstärke der Manschette 2 im proximalen Bereich 5 erreicht hat.

Der Innendurchmesser der Manschette 2 im Übergangsbereich 7 ist größer als in den sich auf beiden Seiten anschließenden Bereichen, nämlich dem proximalen Bereich 5 und dem Endbereich 8. Dieser Endbereich 8 bildet zwischen dem Wulst 11 und dem Wulst 10 am distalen Rand eine Art Zylinderrohrsegment. Der Außendurchmesser der Manschette 2 im Endbereich verringert sich geringfügig zum Wulst 11 hin.

Der insbesondere aus der Figur 2 ersichtliche Hohlkörper 3 besitzt einen proximalen Zylinderrohrabschnitt 16, dessen proximaler Rand als ein sich nach radial außen erstreckender Wulst 17 ausgebildet ist. Dieser Wulst 17 kommt im zusammengebauten Zustand (man vergleiche Figur 1) in der Rinne 12 im Übergangsbereich 7 der Manschette 2 zu liegen. Der Endbereich 8 liegt an der Außenmantelfläche des Zylinderrohrabschnitts 16 des Hohlkörpers 3 an. Bedingt dadurch, dass der Wulst 17 in die Rinne 12 eingreift, ist es nur schwer möglich, die Manschette 2, wenn sie mit dem Endbereich 8 auf den Hohlkörper 13 aufgeschoben ist, durch Auseinanderziehen von dem Hohlkörper 3 zu entfernen.

Zum Anlegen der erfindungsgemäßen Verbindungs-vorrichtung wird die Manschette 2 ausgehend von dem Rand 9 in Richtung des Hohlkörpers aufgerollt. Der aufgerollte Rand 9 wird dann zum Schluss über den Wulst 11 im Übergangsbereich 7 "hinübergezogen". Bedingt dadurch, dass der Außendurchmesser der Manschette 2 im Endbereich 8 geringer ist als im Übergangsbereich 7 verbleibt der hinübergezogene Rand zwischen dem Wulst 11 und dem Wulst 10 am distalen Rand des Endbereiches 8.

In diesem Zustand ist die erfindungsgemäße Verbindungsvorrichtung "einsatzbereit".

Um die Verbindungsvorrichtung 1 mit einem Penis zu verbinden, wird der aufgerollte Rand 9 auf einen eingeführten Penis (die Eichel befindet sich innerhalb des Hohlkörpers 3) in entgegengesetzter Weise wie oben beschrieben auf den Penisschaft abgerollt.

Der Hohlkörper 3 ist an seinem distalen Ende als Kuppel 18 ausgebildet. An der Spitze der Kuppel 18 geht diese in einen Zylinderrohrabschnitt 19 über, der durch eine Scheibe 20 verschlossen ist, die mehrere Öffnungen, nämlich eine zentrale Öffnung 21 und mehrere dezentrale bzw. außermittige Öffnungen 31, besitzt.

In die zentrale Öffnung 21 ist ein Zylinderstift 22 eingesetzt, der axial außen mit einer flachen flexiblen Kappe 23 verbunden ist. Diese Kappe 23 stellt einen Ventildeckel dar und ist aus einem elastischen Material, beispielsweise Gummi gefertigt. Der Stift 22 erstreckt sich durch die zentrale Öffnung 21 hindurch. Auf der der Kappe 23 gegenüberliegenden Seite der Scheibe 20 besitzt der Stift 22 eine nach radial außen vorspringenden Rand 24, der eine Bewegung der Kappe 23 bzw. des Stiftes 22 in distale Richtung verhindert. Die Scheibe 20 bildet zusammen mit der Kappe 23 ein Rückschlagventil; die im Inneren des Hohlkörpers 3 befindliche Luft kann lediglich nach axial außen und somit vom Hohlkörper 3 weg bzw. aus dem Hohlkörper 3 heraus in distaler Richtung entweichen bzw. sich bewegen. Durch den Luftstrom wird die flexible Kappe 23 angehoben, die Luft kann durch die Öffnungen 31 und auch 21, welche radial innen bezüglich des Außenrandes der Kappe 23 angeordnet sind, entweichen. Bewegt sich die Luft in umgekehrter Richtung, verschließt die Kappe 23 die Öffnungen 21 und 31.

Die beschriebenen Teile dieses Rückschlagventils sind aus geeigneten Materialien gefertigt und verfügen über die erforderliche Flexibilität, um der gestellten Aufgabe gerecht werden zu können.

Der Zylinderrohrabschnitt 19 besitzt an seinem distalen Ende einen sich nach radial außen erstreckenden Wulst 26. Auf den Zylinderrohrabschnitt 19 ist im zusammengebauten Zustand eine Vakuumpumpe 4 aufgesetzt. Diese besitzt einen zylinderförmigen Faltenbalg 27. Der Faltenbalg 27 geht an seinem proximalen Ende in einen Zylinderrohrabschnitt 28 über, der im zusammengebauten Zustand außen um den Zylinderrohrabschnitt 19 des Hohlkörpers 3 anliegt.

Der Faltenbalg 27 ist aus einem elastischen Material, beispielsweise einem gummiartigen Material gefertigt, so dass er in Axialrichtung zusammengedrückt werden kann und sich selbstständig wieder ausdehnt. Der Faltenbalg 27 geht an seinem distalen Ende in einen in etwa hohlzylindrischen Abschnitt 29 über, dessen Innendurchmesser geringer ist als der Innendurchmesser des Hohlkörpers 3 und auch des Faltenbalgs 27. In diesen zylindrischen Abschnitt 29 ist ein zweites Rückschlagventil (man vergleiche insbesondere Figur 1) eingesetzt, das in etwa ebenso ausgebildet ist wie das Rückschlagventil in dem distalen Ende des Hohlkörpers 3 und das aus einem Zylinderrohrabschnitt 19', einer Scheibe 20', mehreren Öffnungen 21' bzw. 31', einer Kappe 23' und einem Stift 22' mit einem Vorsprung 24' besteht. Dieses Rückschlagventil ist aus dem gleichen Material gefertigt wie das Rückschlagventil am distalen Ende des Hohlkörpers 3.

Der Zylinderrohrabschnitt 19' besitzt radial außen einen umlaufenden Wulst 25, der, wenn das Rückschlagventil in den hohlzylindrischen Abschnitt 29 eingesetzt ist, in eine damit korrespondieren umlaufende Rinne 30 eingreift.

Beim Zusammendrücken des Faltenbalg 27 in axialer Richtung sowie in proximaler Richtung öffnet sich das Rückschlagventil im hohlzylindrischen Abschnitt 29, während das Rückschlagventil im Hohlkörper 3 geschlossen wird, so dass Luft aus dem Inneren des Faltenbalgs 27 entweicht. Der Bereich oberhalb bzw. distal außen von der Kappe 23' steht mit der Umwelt bzw. der Atmosphäre zum Beispiel über einen Kanal (nicht gezeigt) in Verbindung.

Aufgrund der dem Faltenbalg 27 innewohnenden Elastizität dehnt sich dieser wieder in Axialrichtung und somit in distaler Richtung aus und erzeugt auf diese Weise ein Vakuum verglichen zum Innenraum im Hohlkörper 3. Durch den Überdruck im Inneren des Hohlkörpers 3 wird das in diesem Hohlkörper 3 befindliche Rückschlagventil geöffnet. Luft strömt in Richtung des Inneren des Faltenbalgs 27. Je öfter dieser Pumpvorgang wiederholt wird, desto größer wird das im Inneren des Hohlkörpers 3 erzeugte Vakuum.

Das Innere des Faltenbalgs 27 steht als Reserve-Vakuumraum zur Verfügung. Dringt bei zusammengedrücktem Faltenbalg 27 Luft von außen in das Innere des Hohlkörpers 3 und/oder des Faltenbalgs 27, dann bleibt das Vakuum in etwa erhalten, da und solange sich der Faltenbalg 27 ausdehnen kann. Zudem kann der Benutzer den oben geschilderten Pumpvorgang je nach Bedarf wiederholen.

Natürlich sind außer der beschriebenen Vakuumpumpe 4 mit dem Faltenbalg 27 auch andere per se bekannte Vakuumpumpen mit dem Höhlkörper 3 über die Öffnung(en) 31 verbindbar; auch in diesem Fall steht der Hohlkörper 3 über die Öffnung(en) 31 wie beim Faltenbalg 27 in Wirkverbindung mit der Vakuumpumpe. Ferner sind auch andere Vakuumpumpen einsetzbar, die auf andere Weise mit dem Inneren des Hohlkörpers 3 verbunden sind.

Die Kupplung der erfindungsgemäßen Verbindungsvorrichtung 1 mit einem nicht gezeigten Penisextensionsgerät kann auf beliebige Weise erreicht werden. Beispielsweise kann dazu eine Schlaufe, Schelle oder Ähnliches, die am Zylinderrohrschnitt 28 oder 19 angreift und einen Zug in Richtung des Penisextensionsgerätes auf die erfindungsgemäße Verbindungs-vorrichtung 1 ausübt, eingesetzt werden.

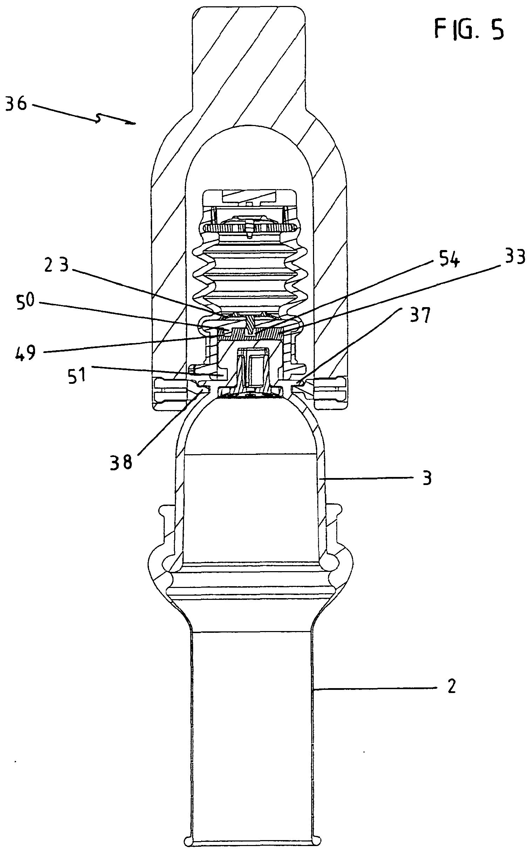

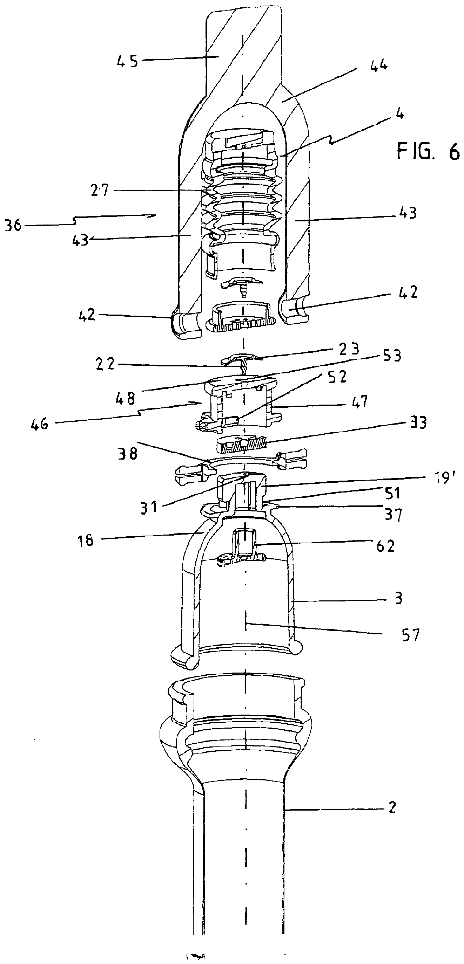

Vorzugsweise dient dazu ein Kupplungselement 36, das in den Figuren 5 und 6 gezeigt ist. Diese Figuren 5 und 6 zeigen zwar eine weitere Ausführungsform der erfindungsgemäßen Verbindungsvorrichtung 1 gemäß den Figuren 1-4. Gleichwohl kann dieses Kupplungselement 36 auch bei der in diesen Figuren 1-4 gezeigten Ausführungsformen zur Anwendung gelangen.

Bei den Figuren 5-8 sind im Übrigen Bezugszeichen für Elemente, welche auch schon bei der in den Figuren 1-4 gezeigten Ausführungsformen verwirklicht sind, mit gleichen Bezugszeichen versehen.

Zur Verbindung des Hohlkörpers 3 mit dem Kupplungselement 36 ist am distalen Ende des Hohlkörpers 3 ein außen radial umlaufender Kranz 37 vorgesehen, der von einem Ring 38 hintergriffen wird. Dieser Ring 38 ist nicht geschlossen, sondern offen, sodass er axial in proximaler Richtung auf den Hohlkörper 3 unter elastischer Aufweitung aufgesetzt werden und den genannten Kranz 37 hintergreifen kann.

Der Ring 38 ist mit zwei sich radial erstreckenden Zapfen 39 versehen, die diametral einander gegenüberliegend mit dem Ring 38 verbunden bzw. einstückig geformt sind. Diese Zapfen 39 erstrecken sich nach radial außen und somit senkrecht zur Längsachse 57 der Verbindungsvorrichtung 1. Diese Zapfen 39 besitzen vier Zapfenelemente 40, die mit einer radial innenliegenden Basis 41 verbunden sind. Die vier Zapfenelemente 40 eines Zapfens 39 sind voneinander beabstandet und bilden zusammen mit den zwischen ihnen liegenden Hohlräumen bzw. Abständen eine Art zylindrischen Bolzen, der jedoch aufgrund der Abstände der Zapfenelemente 40 voneinander zusammengedrückt werden kann.

Auf die beiden Zapfen 39 wird jeweils eine Lagerbuchse 42 eines Bügelarms 43 aufgeschoben. Die Zapfen 39 ragen in das dazugehörige Innere der Lagerbuchse 42 hinein und bewirken eine drehbare Lagerung der Bügelarme 43.

Die Zapfen 39 sind im Übrigen radial außen verdickt. Diese Verdickungen kommen in einer radial außen liegenden Ausnehmung in den Lagerbuchsen 42 zu liegen und stellen damit sicher, dass die Bügelarme 43 bzw. die Lagerbuchsen 42 nur unter Kraftaufwand in radialer Richtung und somit in Richtung der Zapfen 39 von letzteren abgezogen werden können.

Die Bügelarme 43 erstrecken sich in distaler Richtung parallel zum Faltenbalg 27 bzw. zur Vakuumpumpe 4 und sind an ihrem distalen Ende durch einen Bogen 44 miteinander verbunden. Daraus ergibt sich ein in etwa U-förmiges Element, das derart schwenkbar ist, dass die Vakuumpumpe 4 beim Schwenken quasi durch die durch die U-Form umrissene Öffnung durchschwingt.

An dem distalen Ende ist am Bogen 44 eine Einrichtung 45 vorhanden, mittels derer eine Verbindung zu einer Penisextensionsvorrichtung hergestellt werden kann. Bei der gezeigten Ausführungsform handelt es sich bei dieser Einrichtung 45 um einen Clip, der unter Einrasten in eine Muffe gesteckt werden kann. Derartige Einrichtungen sind bekannt. Die Bügelarme 43 zusammen mit dem Bogen 44 und den Lagerbuchsen 42 bilden zusammen eine Art Kupplungselement 36.

Bei den Bügelarmen 43 handelt es sich bei der gezeigten Ausführungsform um streifenartige Bügelarme. Das Querschnittsprofil der Bügelarme 43 kann jedoch beliebig sein und insbesondere elliptisch sein.

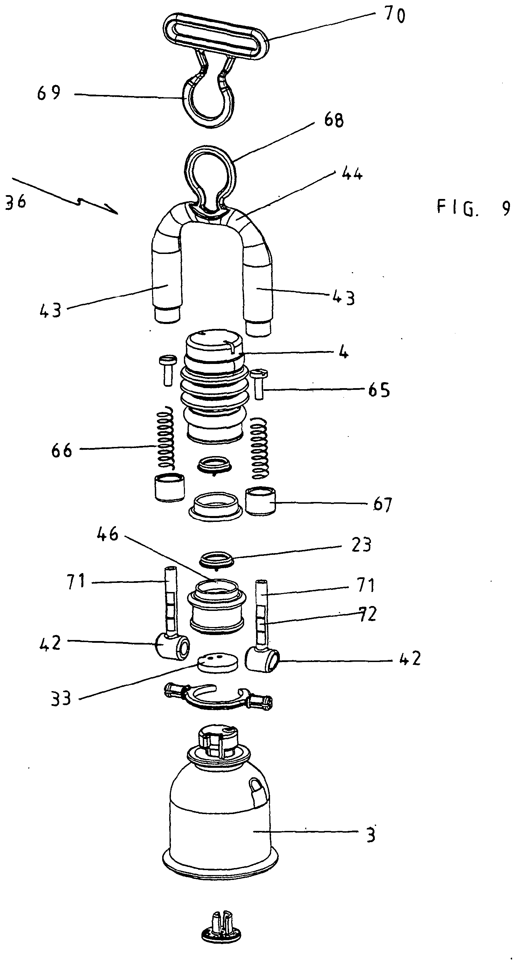

In einen Bügelarm oder in beide Bügelarme 43 kann eine Zugmessvorrichtung eingebaut sein, welche dem Benutzer bei an den Penis angelegter Verbindungsvorrichtung, die zudem unter Zug mit der Penisextensionsvorrichtung verbunden ist, die ausgeübte Zugkraft anzeigt, zum Beispiel mit farbigen Markierungen. Bei einer derartigen Zugmessvorrichtung kann es sich beispielsweise um eine Federwaage handeln. Eine bevorzugte Zugmessvorrichtung ist weiter unten im Zusammenhang mit der Figur 9 näher erläutert.

Der Hohlkörper 3 bei der in den Figuren 5-8 gezeigten Ausführungsform ist an seinem distalen Ende ebenso wie bei den in den Figuren 1-4 gezeigten Ausführungsform als Kuppel 18 ausgestaltet. Diese setzt sich in distaler Richtung in Form eines Zylinderrohrabschnitts 19' fort, der durch eine Scheibe 20' verschlossen ist, welche eine Art Decke bildet. In dieser Decke ist eine durchgehende dezentrale Öffnung 31 bzw. Bohrung vorgesehen. Auf diesem Zylinderrohrabschnitt 19' ist ein topfförmiger Aufsatz 46 mit einer zylindrisch umlaufenden Seitenwand 47 und einem Boden 48 gestülpt. Zwischen dem Boden 48 und der Scheibe 20' liegt eine kreisförmige drehbare Dichtungsscheibe 33 aus einem elastischen oder gummiartigen Material.

Die drehbare Dichtungsscheibe 33 weist auf ihrer distalen Seite (in den Figuren 5 und 6 oben) mehrere Sacklöcher 49 auf, in die jeweils ein Zapfen 50 eingreift, der sich von der proximalen Seite des Bodens 48 axial in proximaler Richtung (in den Figuren 5 und 6 nach unten) erstreckt. Dadurch wird gewährleistet, dass die Dichtungsscheibe 33 gedreht wird, wenn der topfförmige Aufsatz 46 gedreht wird. Die Dichtungsscheibe 33 liegt mit ihrer radialen Seitenwand an der gegenüberliegenden Innenmantelfläche des topfförmigen Aufsatzes 46 an.

Um sicherzustellen, dass der topfförmige Aufsatz 46 gegenüber den Zylinderrohrabschnitt 19' drehbar ist, sind an der Innenfläche der Seitenwand 47 des topfförmigen Aufsatzes 46 zwei sich radial gegenüberliegende sowie nach radial innen erstreckende Vor-sprünge 52 vorgesehen, die in eine sich radial erstreckende Nut 51 am proximalen Ende des Zylinderrohrabschnitts 19' eingreifen. Es kann auch nur ein derartiger Vorsprung vorgesehen sein. Beim Drehen des topfförmigen Aufsatzes 46 gleiten die Vorsprünge 52 in dieser Nut 51 entlang. Beim Drehen des topfförmigen Aufsatzes 46 wird natürlich auch die Dichtungsscheibe 33 entsprechend mitgenommen und gedreht, die distal (in den Figuren oben) auf der Scheibe bzw. dem Deckel 20' aufliegt.

Um den Aufsatz 46 auf den Zylinderrohrabschnitt 19' von oben her aufschieben zu können, sind außen an dem Zylinderrohrabschnitt 19' zwei sich axial erstreckende Nuten 53 ausgenommen. Beim Aufsetzen werden die Vorsprünge 52 soweit gedreht, bis sie in diesen Nuten 53 axial entlang gleiten können. Sobald sich die Vorsprünge auf der axialen Höhe der radialen Nut 51 befinden, kann der Aufsatz 46 bezüglich des Zylinderrohrabschnitts 19' verdreht werden und ist dann nicht mehr in Axialrichtung abziehbar. Soll der Aufsatz wieder abgezogen werden, wird er in eine Stellung verdreht, in der die Vorsprünge 52 axial zur Nut 53 ausgerichtet sind. Um zu erreichen, dass eine Verdrehung nur in eine Richtung erfolgen kann, setzt sich ein Rand der axialen Nut 51 im Bereich der radial erstreckenden Nut 51 in Form eines axialen Steges 64 fort.

Im Boden 48 ist eine durchgehende zentrische Bohrung 48 vorgesehen, durch welche sich ein Stift 22 einer Kappe 23 bis in ein zentrisches Sackloch 54 in der Dichtungsscheibe 33 erstreckt. Diese Kappe 23 sowie der Stift 22 sind ebenso ausgebildet, wie bei der in den Figuren 1 - 4 beschriebenen Ausführungsform.

Der topfförmige Aufsatz 46 mit dem dazugehörigen Boden 48 und der Kappe 22 stellt ein erstes Rückschlagventil dar, das dem ersten Rückschlagventil der in den Figuren 1-4 gezeigten Ausführungsform in etwa entspricht. Dieses Rückschlagventil ist mit einem Faltenbalg 27 und somit einer Vakuumpumpe 4 verbunden. Die Vakuumpumpe 4 ist ansonsten ebenso ausgestaltet, wie die Vakuumpumpe 4 der in der Figur 1-4 gezeigten Ausführungsform. Auch das zweite Rückschlagventil, das in dieser Vakuumpumpe 4 vorhanden ist, ist auf die gleiche Weise aufgebaut, sodass sich weitere Erörterungen erübrigen und aus Gründen der besseren Darstellbarkeit weggelassen werden. Gleiches gilt für die Bezugszeichen.

Durch Drehen des Faltenbalgs 27 werden somit auch der topfförmige Aufsatz 46 sowie die Dichtungsscheibe 33 gedreht.

Die Dichtungsscheibe 33 besitzt ferner eine durchgehende dezentrale Bohrung 55 sowie eine weitere dezentrale durchgehende Belüftungsbohrung 56. Die Bohrung 55 und auch die Belüftungsbohrung 56 sind vom Mittelpunkt der Dichtungsscheibe 33 in radialer Richtung beabstandet. Der radiale Abstand zum Zentrum der Dichtungsscheibe 33 ist dabei der gleiche. Dieser radiale Abstand entspricht dem radialen Abstand der Bohrung 31 in der Scheibe 20'. Mit anderen Worten, der Abstand von der Längsachse 57 zu den Bohrungen 31, 55 und 56 ist der gleiche.

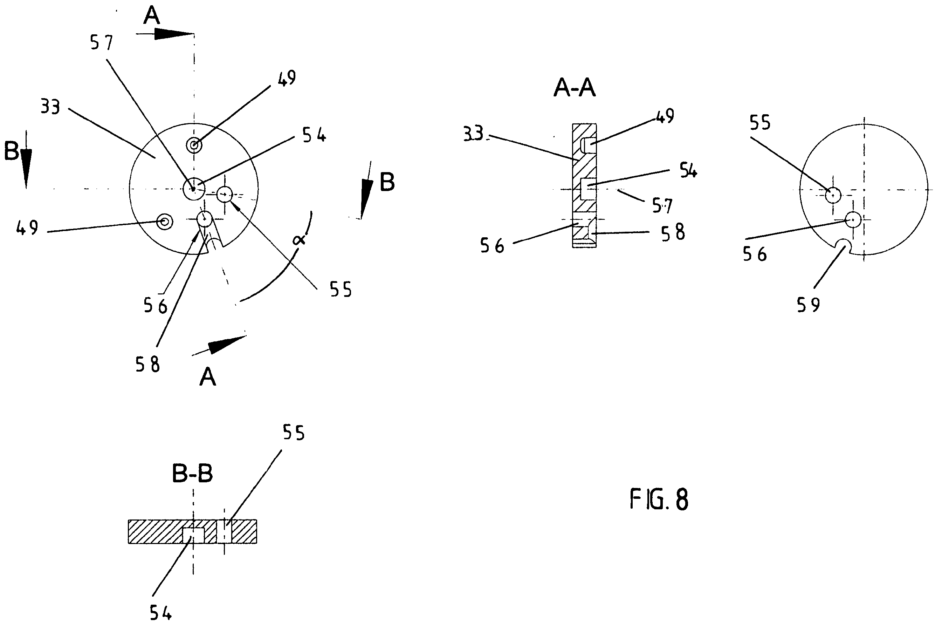

Der Winkel α, der von dem Radius von der Längsachse 57 zur dezentralen Bohrung 55 und von dem Radius von der Längsachse 57 zur dezentralen Belüftungsbohrung 56 in der Dichtungsscheibe 33 eingeschlossen ist, beträgt ca. 60°. Dieser Winkel α wird in der Figur 8 in der links oben gezeigten Aufsicht von den Schnittlinien A und B eingeschlossen.

Durch Drehen der Dichtungsscheibe 33 kann entweder die Bohrung 55 oder die Belüftungsbohrung 56 in axialer Ausrichtung zu der Bohrung 31 gebracht werden.

Befindet sich die Bohrung 55 in axialer Ausrichtung zu der Bohrung 31 und somit über letzterer, dann wird ein durchgehender Kanal vom Innenraum des Hohlkörpers 3 zu dem Innenraum des Faltenbalgs 27 und somit der Vakuumpumpe 4 gebildet. Diese Stellung stellt die Pumpstellung dar, in der bei Betätigung des Faltenbalgs 27 bzw. der Vakuumpumpe 4 Luft aus dem Hohlkörper 3 abgepumpt wird.

Befindet sich die Belüftungsbohrung 56 in axialer Ausrichtung zu der Bohrung 31, dann entspricht dies der Belüftungsstellung, in der der Innenraum des Hohlkörpers 3 mit der Umwelt und somit der Atmosphäre verbunden ist.

Um eine derartige Verbindung zu gewährleisten, erstreckt sich von der distalen Seite der Belüftungsbohrung 56 ein Kanal 58 in radialer Richtung, man vergleiche insbesondere den Schnitt A-A der Figur 8.

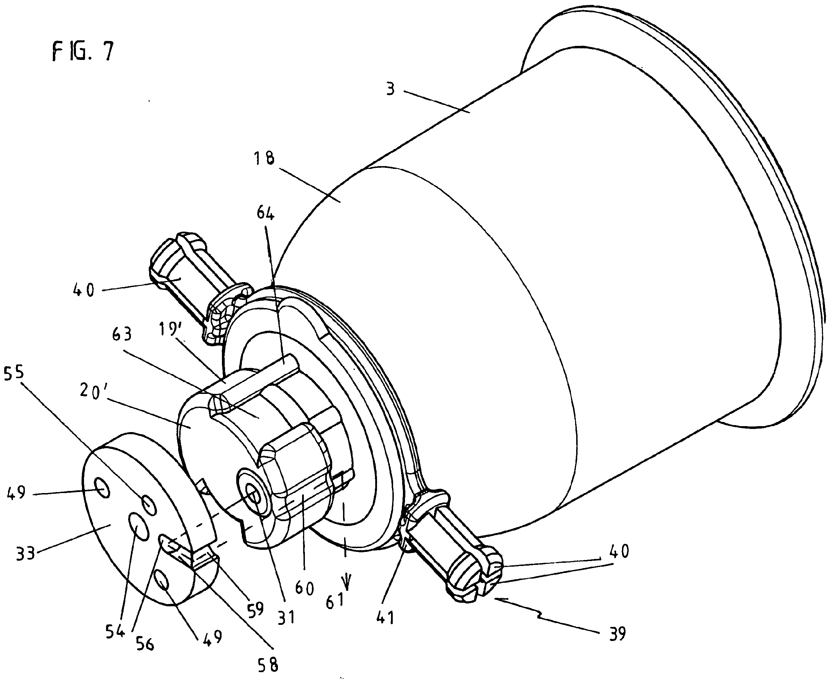

Radial außen geht der sich radial nach außen erstreckende Kanal 58 in eine sich axial erstreckende, nach radial außen offene Nut 59 über, die sich über die gesamte Höhe der Dichtungsscheibe 33 erstreckt, man vergleiche auch Figur 7.

Befindet sich die Belüftungsbohrung 56 in axialer Ausrichtung zur Öffnung 31 und somit direkt darüber, dann befindet sich unterhalb der Dichtungsscheibe 33 und somit in proximaler Richtung eine Nut 60, die in der Außenwand des Zylinderrohrabschnitts 19 ausgebildet ist und sich in axialer Richtung erstreckt. Die Nut 60 mündet somit in der Umwelt bzw. im Freien. Der Weg, der ins Freie führt und über den somit der Innenraum des Hohlkörpers 3 mit der Außenwelt verbunden ist (betrifft diese Belüftungsstelle) ist in der Figur 7 mit einem Pfeil 61 kenntlich gemacht.

Wird die Dichtungsscheibe 33 derart gedreht, dass weder die Bohrung 55 noch die Belüftungsbohrung 56 oberhalb der Bohrung 31 liegt, dann ist diese Bohrung 31 verschlossen. Diese Verschlussstellung wird gewählt, nachdem das gewünschte Vakuum im Inneren des Hohlkörpers 3 mit Hilfe der Vakuumpumpe 4 erreicht worden ist. Am Ende des Pumpvorganges wird dann lediglich die Dichtungsscheibe 33 in diese Verschlussstellung gedreht. Dann steht auch der Innenraum der Vakuumpumpe 4 bzw. das Faltenbalg 27 nicht mehr mit dem Innenraum des Hohlkörpers 3 in Verbindung.

In den Innenraum des Zylinderrohrabschnitts 19' ist ein siebartiger Einsatz 63 eingesetzt, der zum Schutz der Penisspitze dient. Dieser Einsatz kann aus einem weichen Material, jedoch auch aus einem starren Kunststoffmaterial gefertigt sein.

Die in der Figur 9 in Explosionsdarstellung gezeigte Verbindungseinrichtung entspricht in vielerlei Hinsicht der in der Figur 6 gezeigten Verbindungsvorrichtung. Daher sind gleiche Teile bzw. Elemente auch mit den gleichen Bezugszeichen versehen. Der Hohlkörper 3, die Vakuumpumpe 4 sowie der topfförmige Aufsatz 46 mit der dazugehörigen drehbare Dichtungsscheibe 33 sind bei der in der Figur 9 gezeigten Verbindungseinrichtung ebenso ausgestaltet, wie bei der in der Figur 6 gezeigten Verbindungsvorrichtung und auch mit den gleichen Bezugszeichen versehen.

Der Unterschied bei der in der Figur 9 gezeigten Verbindungsvorrichtung liegt in der Einrichtung 45, mittels derer eine Verbindung zu einer nicht gezeigten Penisextensionsvorrichtung hergestellt werden kann.

Die Einrichtung 45 bei der in der Figur 9 gezeigten Ausführungsform besitzt einen Bogen 44 und zwei sich parallel erstreckende Bügelarme 43, die innen hohl sind. Auf die freien Enden dieser hohlen Bügelarme 43 ist jeweils eine Kappe 67 aufgesetzt bzw. aufgeschraubt. Diese Kappen 67 stellen somit den Abschluss der hohlen Bügelarme 43 dar, sind jedoch mit einer zentralen Bohrung (nicht gezeigt) versehen, durch die sich jeweils ein Hohlzylinderstift 71 von außen nach innen erstreckt. Im Inneren der Bügelarme 43 sind jeweils eine Gewindeschraube 65 und eine Feder 66 angeordnet. Der Gewindestift dieser Gewindeschraube 65 ragt in die Feder hinein; eine Seite der Feder 66 stützt sich am Kopf der Gewindeschraube 65 ab, während die andere Seite der Feder 66 auf dem Boden der Kappe 67 abgestützt ist. Die Kappen 67 besitzen in ihrem Boden jeweils eine Bohrung, durch die sich der Hohlzylinderstift 71 von außen nach innen hindurch in die Feder 66 hinein erstreckt.

Der Gewindestift der Gewindeschraube 65 wird in den dazugehörigen Hohlzylinderstift 71 eingeschraubt, der dazu über ein korrespondierendes Gewinde verfügt.

Im zusammengebauten Zustand ragt der Hohlzylinderstift 71 in den dazugehörigen Bügelarm 43 hinein und ist nach außen hin nicht sichtbar. Mit anderen Worten, die Lagerbuchse 42 liegt an dem freien Ende des Bügelarmes 43 an. Bei Ausübung einer Zugkraft auf das Kupplungselement 36 wird der Hohlzylinderstift 71 mehr oder weniger weit aus dem Bügelarm 43 herausgezogen. Außen auf dem Hohlzylinderstift 71 befindet sich eine Markierung 72, die drei farblich unterschiedliche Bereiche aufweist. Je größer die ausgeübte Zugkraft ist, desto weiter wird der Hohlzylinderstift 71 gegen die Kraft der Feder 66 aus dem Bügelarm 43 herausgezogen. Je mehr dieser Bügelarm 43 herausgezogen wird, desto mehr wird auch von der Markierung 72 von außen her sichtbar. Dadurch wird die ausgeübte Zugkraft angezeigt.

Zum Verbinden des Kupplungselementes 36 mit einem Penisextensionsgerät über einen Gurt oder ähnliches dient ein an dem Bogen 44 befestigtes Ringelement 68, das mit einem Ringelement 69, einer Schnalle 70, welche mit einem nicht gezeigten Gurt verbunden werden kann, unter Herstellung einer Verbindung zusammenwirken kann. Derartige Ringelemente sind bekannt.

Damit die Kappen 67 fest mit den dazugehörigen Bügelarmen 43 verbunden werden können, sind die Kappen 67 auf der Innenseite der radial umlaufenden Seitenwand mit einem Gewinde ausgestattet, welches mit einem Außengewinde auf den freien Enden der Bügelarme 43 zusammenwirkt.

### Bezugszeichenliste

- 1: Verbindungsvorrichtung
- 2: Manschette
- 3: Hohlkörper
- 4: Vakuumpumpe
- 5: proximaler Bereich der Manschette 2
- 6: distaler Bereich der Manschette 2
- 7: Übergansbereich des distalen Bereichs 6
- 8: Endbereich des distalen Bereichs 6
- 9: proximaler Rand des proximalen Bereiches 5
- 10: Wulst am distalen Rand des Endbereichs
- 11: Wulst im Übergangsbereich 7 des distalen Bereichs 6
- 12: Rinne
- 13: Innenwandung im Übergangsbereich 7
- 14: zweite Rinne
- 15: Innenwulst
- 16: proximaler Zylinderrohrabschnitt im Endbereich
- 17: Wulst des Hohlkörpers 3
- 18: Kuppel
- 19: Zylinderrohrabschnitt
- 20, 20': Scheibe
- 21,21': Öffnung
- 22, 22': Stift
- 23, 23': Kappe
- 24, 24': Vorsprung
- 25: Wulst
- 26: Wulst
- 27: Faltenbalg
- 28: Zylinderrohrabschnitt
- 29: zylindrischer Abschnitt
- 30: Rinne
- 31,31': dezentrale Öffnung/Bohrung
- 32: durchgehende Öffnung
- 33: drehbare Dichtungsscheibe
- 34: weitere durchgehende Öffnung
- 35: Kanal
- 36: Kupplungselement
- 37: Kranz
- 38: Ring
- 39: Zapfen
- 40: Zapfenelement
- 41: Basis
- 42: Lagerbuchse
- 43: Bügelarm
- 44: Bogen
- 45: Einrichtung
- 46: topfförmiger Aufsatz
- 47: Seitenwand des Aufsatzes 46
- 48: Boden des Aufsatzes 46
- 49: Sackloch in Dichtungsscheibe 33
- 50: Zapfen am Boden 48
- 51: Nut
- 52: Vorsprung
- 53: durchgehende Bohrung im Boden 48
- 54: zentrales Sackloch in Dichtungsscheibe 33
- 55: durchgehende dezentrale Bohrung in Dichtungsscheibe 33
- 56: dezentrale Belüftungsöffnung der Dichtungsscheibe 33
- 57: Längsachse
- 58: Kanal
- 59: Nut in Dichtungsscheibe 33
- 60: Nut in Zylinderrohrabschnitt 19'
- 61: Pfeil
- 62: siebartiger Einsatz
- 63: Axiale Nut
- 64: Axialer Steg
- 65: Schraube
- 66: Feder
- 67: Kappe
- 68: Ringelement
- 69: Ringelement
- 70: Schnalle
- 71: Hohlzylinderstift
- 72: Markierung

## Patentansprüche

1. Verbindungsvorrichtung für den distalen Teil eines Penis mit einer Penisextensionsvorrichtung, wobei die Verbindungsvorrichtung (1) einen am proximalen Ende offenen, länglichen, starren Hohlkörper (3), der zur Aufnahmen des distalen Endes des Penis und zur Befestigung an der Penisextensionsvorrichtung dient, und an seinem distalen Ende eine Öffnung (31) besitzt, die in Wirkverbindung mit einer Vakuumpumpe (4) steht oder in eine derartige Wirkverbindung gebracht werden kann,
und eine elastische, schlauchartige Manschette (2) zur Anlage mit ihrem proximalen Bereich (5) an den Penisschaft, die im zusammengebauten Zustand der Verbindungsvorrichtung (1) mit ihrem distalen Bereich (6) das proximale Ende des Hohlkörpers (3) umschließt und außen an ihm anliegt, aufweist,
**dadurch gekennzeichnet, dass**
zwischen der Öffnung (31) und der Vakuumpumpe (4) ein Dreiwegeventil angeordnet ist, das folgende Stellungen einnehmen kann:
i) der Innenraum des Hohlkörpers (3) ist mit der Umwelt verbunden,
ii) die Öffnung (31) und somit der Hohlkörper (3) sind verschlossen und
iii) der Innenraum des Hohlkörpers (3) ist mit der Vakuumpumpe (4) verbunden.

2. Verbindungsvorrichtung nach Anspruch 1,
**dadurch gekennzeichnet, dass**
das Dreiwegeventil eine drehbare Dichtungsscheibe (33) aufweist, in der mindestens eine durchgehende Öffnung (32) vorgesehen ist, die dezentral angeordnet ist und durch Drehen der Dichtungsscheibe in Ausrichtung mit der Öffnung (31) gebracht werden kann und
dass in der Dichtungsscheibe (33) eine weitere durchgehende Öffnung (34) vorgesehen ist, die ebenfalls dezentral angeordnet ist und durch Drehen der Dichtungsscheibe (33) in axiale Ausrichtung mit der Öffnung (31) gebracht werden kann, und die über einen Kanal (58) mit der Umwelt in Verbindung steht und
die Dichtungsscheibe (33) derart gedreht werden kann, dass die Öffnung (31) verschlossen ist.

3. Verbindungsvorrichtung nach Anspruch 2,
**dadurch gekennzeichnet, dass**
die Wanddicke der Manschette (2) im distalen Bereich (6) größer ist als im proximalen Bereich (5).

4. Verbindungsvorrichtung nach Anspruch 2 oder 3,
**dadurch gekennzeichnet, dass**
die Wanddicke der Manschette (2) im distalen Bereich (6) 4 bis 15 mal so stark ist wie die Wanddicke der Manschette (2) im proximalen Bereich (5).

5. Verbindungsvorrichtung nach einem der vorhergehenden Ansprüche,
**dadurch gekennzeichnet, dass**
die Wanddicke der Manschette (2) im proximalen Bereich (5) 0,20 bis 0,40 mm und im distalen Bereich (6) 2 bis 5 mm beträgt.

6. Verbindungsvorrichtung nach einem der vorhergehenden Ansprüche,
**dadurch gekennzeichnet, dass**
sich die Wanddicke der Manschette (2) am Übergang vom distalen Bereich (6) zum proximalen Bereich (5) hin kontinuierlich verringert.

7. Verbindungsvorrichtung nach einem der vorhergehenden Ansprüche,
**dadurch gekennzeichnet, dass**
die Manschette (2) aus medizinischem Silikon gefertigt ist.

8. Verbindungsvorrichtung nach einem der vorhergehenden Ansprüche,
**dadurch gekennzeichnet, dass**
der Hohlkörper (3) zylinderförmig ausgebildet ist,
der distale Bereich (6) der Manschette (2) unterteilt ist in einen Übergangsbereich (7) zum proximalen Bereich (5) und einen dem Hohlkörper (3) zugewandten Endbereich (8),
die Manschette (2) im proximalen Bereich (5) und in dem dem Hohlkörper (3) zugewandten Endbereich (8) zylinderförmig ausgebildet ist und
der Innendurchmesser der Manschette (2) im proximalen Bereich (5) sowie in dem dem Hohlkörper (3) zugewandten Endbereich (8) in etwa gleich sind.

9. Verbindungsvorrichtung nach Anspruch 8,
**dadurch gekennzeichnet, dass**
der Hohlkörper (3) an seinem proximalen Rand einen sich nach radial außen erstreckender Wulst (17) besitzt,
die Manschette (2) sich im Übergangsbereich (7) wulstartig (11) nach radial außen erstreckt und in ihrer Innenwandung (13) eine umlaufende Rinne (12) aufweist, die in etwa flächen- und formkongruent zum Wulst (17) des Hohlkörpers (3) ausgebildet ist.

10. Verbindungsvorrichtung nach einem der vorhergehenden Ansprüche,
**dadurch gekennzeichnet, dass**
das distale Ende des Hohlkörpers (3) kuppelförmig (18) ausgestaltet ist und die Öffnung (31) in der Kuppel (18) angeordnet ist.

11. Verbindungsvorrichtung nach einem der vorhergehenden Ansprüche,
**dadurch gekennzeichnet, dass**
der Hohlkörper (3) an seinem distalen Ende ein Kupplungselement (36) besitzt, über welches die Penisextensions-vorrichtung einen Zug auf die Verbindungseinrichtung (1) übertragen kann.

12. Verbindungsvorrichtung nach Anspruch 11,
**dadurch gekennzeichnet, dass**
das Kupplungselement (36) beweglich und insbesondere schwenkbar an dem Hohlkörper (3) befestigt und gelagert ist.

## Claims

1. Connecting device for the distal part of a penis with a penis extension device wherein the connecting device (1) comprises an elongate, rigid hollow body (3), which is open at the proximal end and is used for receiving the distal end of the penis and for securing on the penis extension device and, at its distal end, has an opening (31) that is in operational connection to a vacuum pump (4) or can be brought into such an operational connection,
and an elastic tubular sleeve (2) which is placed with its proximal area (5) onto the shaft of the penis and which, when the connecting device (1) is in the assembled state, has its distal area (6) enclosing the proximal end of the hollow body (3) and bearing on the outside thereof,
**characterized in that**
a three-way valve is arranged between the opening (31) and the vacuum pump (4) and can assume the following positions:
i) the interior of the hollow body (3) is connected to the environment,
ii) the opening (31) and therefore the hollow body (3) are closed, and
iii) the interior of the hollow body (3) is connected to the vacuum pump (4).

2. Connecting device according to claim 1,
**characterized in that**
the three-way valve comprises a rotatable sealing disk (33), in which at least one continuous opening (32) is provided which is arranged off-center and can be brought into alignment with the opening (31) by rotation of the sealing disk, and that
a further continuous opening (34) is provided in the sealing disk (33), which is likewise arranged off-center and can be brought into axial alignment with the opening (31) by rotation of the sealing disk (33) and is connected to the environment via a channel (58), and
the sealing disk (33) can be rotated in such a way that the opening (31) is closed.

3. Connecting device according to claim 2,
**characterized in that**
the wall thickness of the sleeve (2) in the distal area (6) is greater than in the proximal area (5).

4. Connecting device according to claim 2 or 3,
**characterized in that**
the wall thickness of the sleeve (2) in the distal area (6) is 4 to 15 times as great as the wall thickness of the sleeve (2) in the proximal area (5).

5. Connecting device according to one of the preceding claims,
**characterized in that**
the wall thickness of the sleeve (2) is 0.20 to 0.40 mm in the proximal area (5) and 2 to 5 mm in the distal area (6)

6. Connecting device according to one of the preceding claims,
**characterized in that**
the wall thickness of the sleeve (2) decreases continuously at the transition from the distal area (6) to the proximal area (5).

7. Connecting device according to one of the preceding claims,
**characterized in that**
the sleeve (2) is made from medical silicone.

8. Connecting device according to one of the preceding claims,
**characterized in that**
the hollow body (3) is cylindrical in shape,
the distal area (6) of the sleeve (2) is divided into a transition area (7) to the proximal area (5) and an end area (8) facing the hollow body (3),
the sleeve (2) is designed in the proximal area (5) and in the end region (8) facing the hollow body (3) in a cylindrical form, and
the inner diameter of the sleeve (2) in the proximal area (5) and in the end area (8) facing the hollow body (3) are approximately equal.

9. Connecting device according to caim 8,
**characterized in that**
the hollow body (3) has a bead (17) extending radially outwards at its proximal edge,
the sleeve (2) extends radially outwards in the transition region (7) like a bead (11) and has a circumferential channel (12) in its inner wall (13) which is formed approximately congruently to the bead (17) of the hollow body (3) in terms of surface area and shape.

10. Connecting device according to one of the preceding claims,
**characterized in that**
the distal end of the hollow body (3) is arranged in a dome-shaped (18) shape and the opening (31) is arranged in the dome (18).

11. Connecting device according to one of the preceding claims,
**characterized in that**
the hollow body (3) has a coupling element (36) at its distal end, via which the penis extension device can transfer a pull to the connecting device (1).

12. Connecting device according to claim 11,
**characterized in that**
the coupling element (36) is movable and, in particular, pivotably mounted and supported on the hollow body (3).

## Revendications

1. Dispositif de jonction pour la partie distale d'un pénis avec un dispositif d'extension du pénis ;
selon lequel le dispositif de jonction (1) possède un corps creux (3), rigide, allongé et ouvert au niveau de l'extrémité proximale, lequel sert à la réception de l'extrémité distale du pénis et à sa fixation sur le dispositif d'extension du pénis, ainsi qu'une ouverture (31) au niveau de son extrémité distale qui se trouve en liaison active avec une pompe à vide (4) ou qui peut être mise dans une telle liaison active ; et
selon lequel le dispositif de jonction (1) comprend une manchette (2) élastique, de forme tubulaire, laquelle est destinée à venir se mettre en butée sur le corps de la verge par sa zone proximale (5) et laquelle enserre l'extrémité proximale du corps creux (3) avec sa zone distale (6), quand le dispositif de jonction (1) se trouve à l'état assemblé, et vient en appui à l'extérieur contre le corps creux (3) ;
**caractérisé en ce que**
une soupape à trois voies est disposée entre l'ouverture (31) et la pompe à vide (4), laquelle soupape à trois voies peut adopter les positions suivantes :
i) le compartiment interne du corps creux (3) est relié avec l'extérieur ;
ii) l'ouverture (31) et, par conséquent, le corps creux (3) sont obturés ; et
iii) le compartiment interne du corps creux (3) est relié à la pompe à vide (4).

2. Dispositif de jonction selon la revendication 1,
**caractérisé en ce que**
la soupape à trois voies comprend une rondelle d'étanchéité (33) rotative, dans laquelle est prévue au moins une ouverture (32) continue, laquelle est disposée de manière décentralisée et laquelle peut être amenée en alignement avec l'ouverture (31) par la rotation de la rondelle d'étanchéité (33) ; et
**caractérisé en ce que**
une autre ouverture (34) continue est prévue dans la rondelle d'étanchéité (33), laquelle est également disposée de manière décentralisée et laquelle peut être amenée en alignement axial avec l'ouverture (31) par la rotation de la rondelle d'étanchéité (33) et laquelle se trouve en liaison avec l'extérieur par l'intermédiaire d'un canal (58) ; et
selon lequel la rondelle d'étanchéité (33) peut être tournée de telle sorte que l'ouverture (31) puisse être obturée.

3. Dispositif de jonction selon la revendication 2,
**caractérisé en ce que**
l'épaisseur de la cloison de la manchette (2) est plus importante dans la zone distale (6) que dans la zone proximale (5).

4. Dispositif de jonction selon la revendication 2 ou 3,
**caractérisé en ce que**
l'épaisseur de la cloison de la manchette (2) dans la zone distale (6) est 4 à 15 fois plus importante que l'épaisseur de la cloison de la manchette (2) dans la zone proximale (5).

5. Dispositif de jonction selon l'une des revendications précédentes,
**caractérisé en ce que**
l'épaisseur de la cloison de la manchette (2) est comprise entre 0,20 mm et 0,40 mm dans la zone proximale (5) et est comprise entre 2 mm et 5 mm dans la zone distale (6).

6. Dispositif de jonction selon l'une des revendications précédentes,
**caractérisé en ce que**
l'épaisseur de la cloison de la manchette (2) se rétrécit continuellement au niveau du passage de la zone distale (6) en direction de la zone proximale (5).

7. Dispositif de jonction selon l'une des revendications précédentes,
**caractérisé en ce que**
la manchette (2) est fabriquée en un silicone à usage médical.

8. Dispositif de jonction selon l'une des revendications précédentes,
**caractérisé en ce que**
le corps creux (3) est conçu en forme de cylindre ;
la zone distale (6) de la manchette (2) est divisée en une zone de passage (7) en direction de la zone proximale (5) et en une zone d'extrémité (8) orientée vers le corps creux (3) ;
la manchette (2) est conçue en forme de cylindre dans la zone proximale (5) et dans la zone d'extrémité (8) qui est orientée vers le corps creux (3) ; et
le diamètre intérieur de la manchette (2) dans la zone proximale (5) ainsi que celui dans la zone d'extrémité (8) qui est orientée vers le corps creux (3) sont approximativement identiques.

9. Dispositif de jonction selon la revendication 8,
**caractérisé en ce que**
le corps creux (3) possède, au niveau de sa bordure proximale, un bourrelet (17) qui s'étend vers l'extérieur de manière radiale ;
la manchette (2) s'étend vers l'extérieur de manière radiale dans la zone de passage (7) en formant un bourrelet (11) et comprend dans sa paroi interne (13) une rigole périphérique (12), laquelle est conçue de manière à peu près congruente de par la surface et de par la forme vis-à-vis du bourrelet (17) du corps creux (3).

10. Dispositif de jonction selon l'une des revendications précédentes,
**caractérisé en ce que**
l'extrémité distale du corps creux (3) est configurée en forme de coupole (18) et l'ouverture (31) est disposée dans la coupole (18).

11. Dispositif de jonction selon l'une des revendications précédentes,
**caractérisé en ce que**
le corps creux (3) possède un élément de couplage (36) au niveau de son extrémité distale, par l'intermédiaire duquel le dispositif d'extension du pénis peut transmettre une traction sur le mécanisme de jonction (1).

12. Dispositif de jonction selon la revendication 11,
**caractérisé en ce que**
l'élément de couplage (36) est fixé et positionné de manière mobile et, en particulier, de manière pivotante au niveau du corps creux (3).
